# EUROPEAN PATENT APPLICATION

(11) **EP 3 660 496 A1**
(43) Date of publication of application: **03.06.2020**
(21) Application number: 19211700.0
(22) Date of filing: 27.11.2019
(51) Int. Cl.: G01N 25/72, G01N 27/90

(54) **METHOD FOR NON-DESTRUCTIVE TESTING OF COMPONENTS PRODUCED FROM CARBON FIBER REINFORCED COMPOSITE MATERIALS**

(30) Priority: 29.11.2018 RU 2018142127
(71) Applicant: Obshchestvo S Ogranichennoy Otvetstvennostyu "Kachestvo Nerazrushayushchego Kontrolya", Moscow 127051 (RU)
(72) Inventor: BATOV, Georgy, 127473 Moscow (RU)
(74) Representative: Lapienis, Juozas

(57) **Abstract**

The invention relates to the field of research and analysis of materials, in particular, to methods for testing the integrity of components produced from carbon fiber based composite materials, and can be used to detect manufacturing defects and damages received during service in components produced from carbon fiber reinforced composite materials.

The claimed method for non-destructive testing of carbon fiber reinforced composite materials includes heating the material with an external source, recording the temperature distribution pattern of the tested material, analyzing the recorded temperature distribution pattern and detecting the presence of defective areas, wherein the material is heated by exposing the carbon fiber included in the material to a highfrequency electromagnetic field.

Thus, the claimed invention is a method for non-destructive testing of the components produced from carbon fiber reinforced composite materials which allows for an efficient and reliable assessment of their condition over the entire depth of the material structure without exposing the coating layers to excessive load, if any.

## Description

The invention relates to the field of research and analysis of materials, in particular, to methods for testing the integrity of components produced from carbon fiber based composite materials, and can be used to detect manufacturing defects and damages received during service in components produced from carbon fiber reinforced composite materials.

The use of composite materials is an upcoming trend of modern technology. Carbon fiber reinforced composite materials are widely used in various fields of mechanical engineering, in particular, in the production of vehicles for various purposes - as structural elements of aircraft, river and sea vessels, due to the advantages of these materials over metals and alloys used earlier, such as high corrosion resistance, resistance to cyclic loads, light weight, etc.

Despite the significant advantages described above, carbon fiber reinforced composite materials have a disadvantage. It has low impact resistance leading to material delamination and cracking in service, which have a great impact on component bearing ability.

On the other hand, despite the intensive development of technologies for manufacturing composite materials there has not been reached enough level of production to produce defect-free components. The manufacturing defects of carbon fiber reinforced composite materials include stratification, bad adhesion of matrix and fibers, uneven distribution of carbon fibers, cracks and various other violations of the material continuity.

Thus, to evaluate the condition of a component produced from a composite material in order to determine the appropriateness of its further use, it is necessary to have data both about damage received in service and production imperfections.

To collect this data various non-destructive testing methods known from the general NDT technology, such as radiographic, ultrasound, etc. are used. Nevertheless, it should be taken into account that composite materials have a complex structure and anisotropy of properties. For this reason, many non-destructive testing methods successfully applied, for example, to metal components are not applicable to composite materials. Thus, there is a need to introduce new non-destructive testing methods or to adapt the existing ones.

Nowadays infrared inspection is one of the most promising non-destructive testing methods, which is effectively used for all types of composite materials. To implement this method, the tested material is heated by an external source, then the temperature distribution pattern of the tested material is recorded. The recorded temperature distribution pattern is analyzed to detect the presence of defective areas. The temperature distribution pattern, i.e. the temperature distribution on the surface of the object, includes information about the deviation of the heat transfer process in the tested material depending on its internal structure and the presence of hidden internal defects.

Usually, equipment is used for heating of the tested material, heating the material from the outside, for example, lamps, blow dryers. Examples of such testing methods can be found in the following studies:
1) Murphy John C, Spicer Jane W, Robert Osiander Patent US20060114965A1 Thermal-based methods for nondestructive evaluation
2) Vavilov V.P., Ivanov A.I. Pulse Thermal Testing of Multilayer Articles // Defectoscopy, 1984 No. 6. P. 39-47.
3) Vavilov V.P., Klimov A.G., Shiryaev V.V. Active Thermal Testing of Water in Airframe Cellular Structures // Defectoscopy. 2002. No. 12. P. 32-38.

The disadvantage of this method is that heating is carried out through the surface of the material. Due to the fact that the finished articles in most cases are coated with a paint coating, which, in turn, has low thermal conductivity, it is impossible to transfer a large amount of thermal energy to the article without the risk of overheating, and, as a result, damage to the paint coating. In this case, an excessively heated surface of the material masks thermal anomalies, and if the heating source does not provide proper uniformity of heating of the entire surface of the material, it further creates false indications when recording its temperature distribution pattern.

The closest method for thermal non-destructive testing of composite materials, adopted as a prototype, is the testing method described in the patent document RU 2616438 (publ. 14.04.2017), where the material under examination is treated with a highly wetting electrically conductive fluid, and the material is heated by exposing the fluid to electromagnetic radiation, preferably of the super-high-frequency type. Thus, the liquid, penetrating into microcracks in the material, provides for heating of the material from the inside, which, in turn, allows for obtaining a more detailed temperature distribution pattern map, and, therefore, for better examining the condition of the material under examination. The disadvantage of this method is that the subsequently heated liquid penetrates into the material under examination only through the cracks formed in the surface, and if the integrity of the surface is not violated, and the defect formed at the stage of material manufacturing is located deeply inside, the method offered in this patent source will not allow for effectively detecting the presence of a defective area.

Also in general the same approach to inspection is described in patent document US20060114965A1. Active heating of composite materials with microwaves is described in this document, including the use of emerged conductive wire to analyse the composite properties. (the conductive media reflects microwave radiation). But certainly other frequency diapason is used in US 20060114965A1 and HP 6890B oscillator (5-10 GHz) to produce microwaves at a frequency of 9 GHz wavelength for such frequency is 1-10 sm. In this invention the frequency is 50-500 kHz. So the wavelength is 1000 times longer than microwaves. Of course in both cases it is an electromagnetic radiation, but it like compare X-ray and visible light. The interaction of long waves with media is different from microwaves. The long waves induce the eddy current inside the material and heating of material is caused by electrical resistance. Only conductive fibers are heated. In case of microwaves the nature of heating is molecular friction. In case of long waves we can analyze multilayer materials on great thinness with precise sensitivity, broken fibers greatly change the electrical resistance.

It should be noted that a method for thermal non-destructive testing of electrically conductive materials is also known from the prior art, the method including their heating with electric currents induced by a high-frequency electromagnetic field. In general, induction heating of materials is widely used in various fields of metallurgy. For one, in the patent document US 4480928 a method for thermal non-destructive testing of metal blanks using this type of heating is disclosed, however, it is applicable only for detection of surface defects, because the eddy currents induced in the metal are not able to penetrate deeply into the material, and its high thermal conductivity masks defects in the interior of the material

In patent document US8220991B2 electromagnetically heating a conductive medium in a composite aircraft component is described. Heating of solid metal parts emerged inside the composite media is described. It also does not use internal conductivity of carbon fiber reinforced material.
Thus, the problem to be solved by the present invention is to provide a method for non-destructive testing of the components produced from carbon fiber reinforced composite materials, which allows for an efficient and reliable assessment of their condition over the entire depth of the material structure without exposing the coating layers to excessive load, if any.

The problem set is solved through developing of a method for non-destructive testing of the components produced from carbon fiber reinforced composite materials, which includes heating the material with an external source, recording the temperature distribution pattern of the tested material, analyzing the recorded temperature distribution pattern and detecting the presence of defective areas, wherein the material is heated by exposing the carbon fiber included in the material to a high-frequency electromagnetic field.

The claimed method, like other methods for thermal non-destructive testing of materials known from the prior art, is based on the anisotropy of the thermophysical properties of the object under test, which manifests itself if there is a defect. However, in the developed method, in contrast to the prior art methods mentioned above, it is taken into account that the carbon fiber is able to conduct electric current well and it can be heated when exposed to a high-frequency electromagnetic field due to the eddy currents induced inside. In this case a component from composite materials is heated from the inside. Using this technique the uniformity of heating of the material will depend on the degree of its continuity and homogeneity: on the one hand, any material defect will affect its thermal conductivity, on the other hand, a defect that is somehow related to the deformation of the carbon fiber itself will affect the electrical resistance of the carbon fiber at the defect spot, which will cause its excessive heating in comparison with the defect-free areas. It is important to understand that the temperature difference between the defective and defect-free areas will be affected by both the inequality of their thermophysical properties and the uneven distribution of the density of electric currents. Thus, the developed method allows for achieving the technical effect consisting in efficient and reliable thermal non-destructive testing of carbon fiber reinforced composite materials due to induction heating of the carbon fiber in the composition of the material, recording the temperature distribution pattern of the tested material with subsequent analysis of the recorded temperature distribution pattern in order to detect the presence of defective areas. Moreover, this method will allow for detecting defects of a smaller size, which is due to the physical processes it is based on.

Due to the fact that thermal energy is released inside the material, its path necessary for detecting the presence of defective areas is reduced as compared to the methods using external heating. Here, the thermal energy propagates from inside to outside, where it is recorded, in contrast to the methods where the thermal energy is first distributed from outside to inside the material, and then it returns and is only then recorded, while the excessively heated surface interferes with thermal picture of internal defects.

A further technical effect is also the acceleration of the process of non-destructive testing of a carbon fiber reinforced composite material. An achievement of said technical effect becomes possible due to the possibility of using sources of electromagnetic radiation of high intensity without the danger of overheating the coating.

It should be taken into consideration that the method for non-destructive testing according to the invention is preferably applied to composite materials of a layered or fibrous structure, where the carbon fiber is present in the form of fibers or fabric, however, it is also applicable to composite materials strengthened with particles, although its effectiveness in this case will be much lower.

The use of this method for non-destructive testing, for example, of a carbon fiber reinforced composite material, such as carbon fiber reinforced plastic, is highly effective due to the low thermal conductivity of this material (excessive heating of the defective areas does not normalize for a long time) and its high electrical resistance, which contributes to heat releasing and a large penetration depth of the electromagnetic field.

Carbon fiber of a composite material is preferably heated by a high-frequency electromagnetic field at a frequency of 50-500 kHz. A change in the frequency of the electromagnetic field excited around the inductor acting as a source of electromagnetic radiation affects the depth of its penetration and, therefore, in a preferred embodiment of the present invention is selected for a certain material.

The temperature distribution pattern is recorded by means of a thermal imaging device. The high temperature contrast and surface without marks from overheat during part heating give clear infrared picture which can be received with general infrared equipment used for general NDT purpose.

The claimed method for non-destructive testing of carbon fiber reinforced composite materials may be either one-sided, in which the inductor and means for recording thermal radiation are located on one side of the object under test, or two-sided, in which the inductor and means for recording thermal radiation are located on opposite sides of the object under test.

In case of two-sided inspection the imperfection can be detected immediately without waiting of thermal conductivity heat transfer. If defective area has another conductivity, the amount of electromagnetic energy that reaches the opposite side will depend on integral conductivity of all layer between inductor and infrared camera. The stronger the field is, the faster the material heats, therefore, it is possible to perform inspection with great speed and it is possible to inspect carbon fiber reinforced materials with a thickness of 40 mm.

A further advantage of the claimed method is the ability to test articles of complex shape, which is ensured by the possibility of using an inductor of arbitrary shape, preferably repeating the profile of the object under examination.

The claimed invention is explained in more detail using the following figures:
Fig. 1 is a schematic diagram of physical processes that occur in a defective component produced from a carbon fiber reinforced composite material as a result of its exposure to a high-frequency magnetic field;
Fig. 2 is a schematic diagram of an installation for conducting one-sided thermal non-destructive testing according to one of the preferred embodiments of the claimed method;
Fig. 3 is a schematic diagram of an installation for conducting two-sided thermal non-destructive testing according to another preferred embodiment of the claimed method;
Fig. 4 is an example of an image of the recorded temperature distribution pattern of a tested component produced from a carbon fiber reinforced composite material.

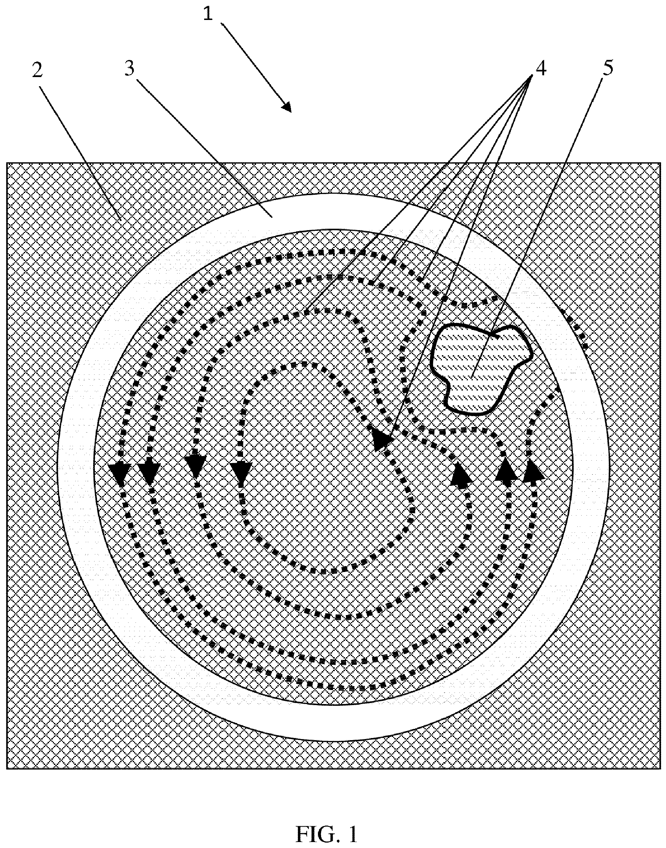

Fig. 1 schematically illustrates physical processes occurring in an example of a defective component produced from a carbon fiber reinforced composite material as a result of its exposure to a high-frequency magnetic field. A component 1 produced from a layered structure composite material comprising a carbon fiber layer 2 is shown in section view. Around the inductor 3 located near the material of the component 1 a high-frequency electromagnetic field is generated, when exposed to which eddy currents 4 are induced in the carbon fiber layer 2 according to the laws of electrodynamics, causing it to heat.

In accordance with the Joule - Lenz law quantifying the electric current thermal effect, the amount of heat released in an area of a circuit per unit time is proportional to the resistance of the area. The defective area 5 of the carbon fiber layer 2 will have greater resistance due to the violation of continuity and, as a consequence, the electrical conductivity of the layer 2 therefore will have more heat when uniformly exposing the layer 2 to electromagnetic field.

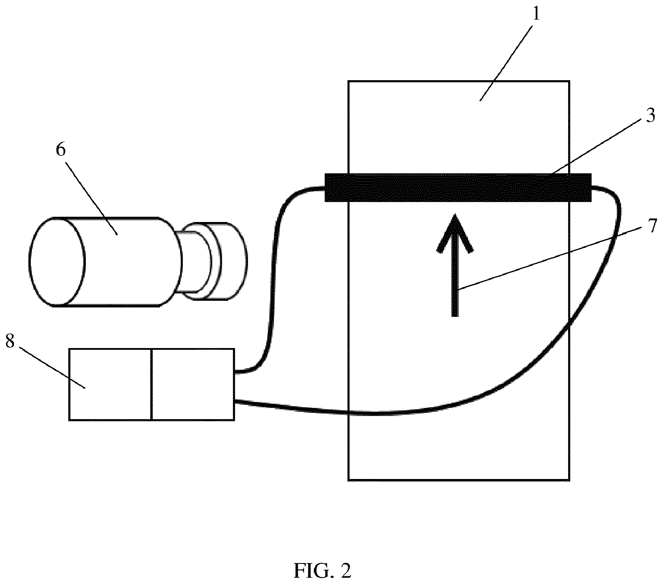

Fig. 2 schematically illustrates an installation for conducting one-sided thermal non-destructive testing according to a preferred embodiment of the claimed method. When conducting one-sided testing, the inductor 3 and the thermal imaging device 6 are placed on one side of the component 1 produced from a carbon fiber reinforced composite material. The material of the component 1 is heated by moving along it in the direction 7 of the inductor 3 around which a high-frequency electromagnetic field is excited by means of an alternating current generator 8. Afterwards, the thermal imaging device 6 records the temperature distribution pattern of the material of the tested component 1 for further analysis thereof and detecting the presence of defective areas.

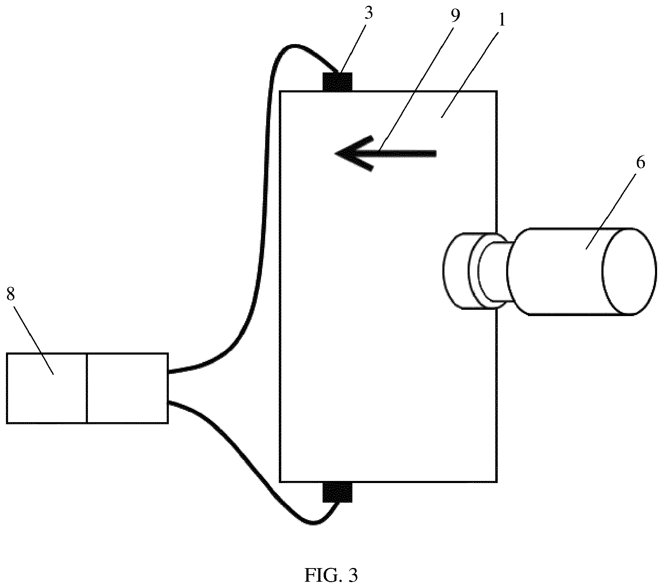

Fig. 3 schematically illustrates an installation for conducting two-sided thermal non-destructive testing according to a preferred embodiment of the claimed method. When conducting two-sided testing, the inductor 3 and the thermal imaging device 6 are placed on different sides of the component 1 produced from a carbon fiber reinforced composite material. Just as when conducting one-sided testing, the material of the component 1 is heated by moving along it in the direction 9 of the inductor 3 around which a high-frequency electromagnetic field is excited by means of an alternating current generator 8. Here, as an example only, the inductor 3 is moved in the direction 9, which is different from the direction 7 presented in the one-sided testing example (Fig. 2). Afterwards, the thermal imaging device 6 records the temperature distribution pattern of the material of the tested component 1 for further analysis thereof and detecting the presence of defective areas.

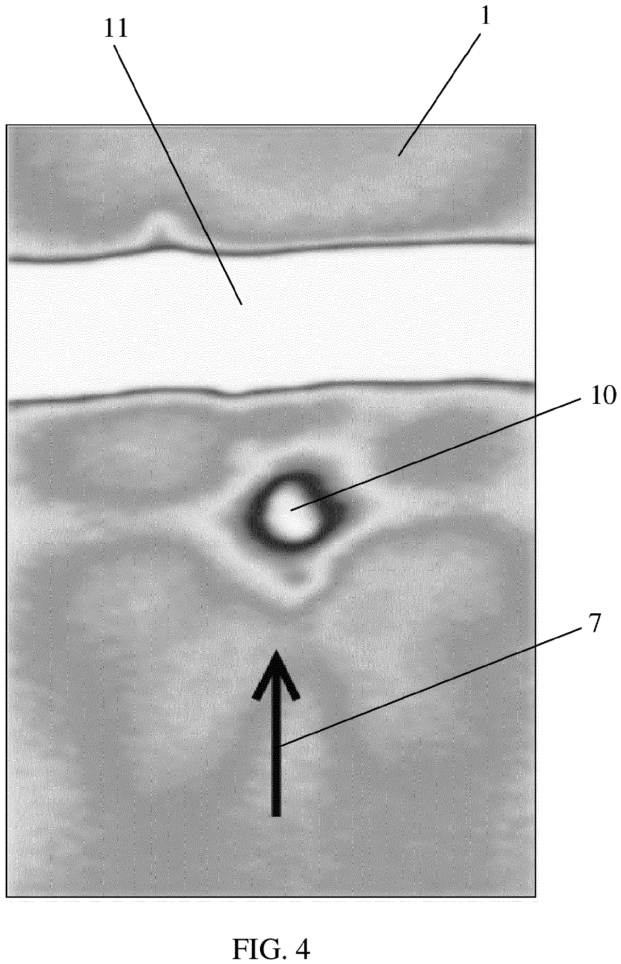

Fig. 4 is an example of an image (thermogram) recorded by a thermal imaging device 6 (not indicated in the figure) of the recorded temperature distribution pattern of a tested component 1 produced from a carbon fiber reinforced composite material. The material under examination of the component 1 is heated by exposure to a high-frequency electromagnetic field of the inductor 3 (not indicated in the figure), which is moved in the direction 7. As can be seen in the presented example, the heated material of the component 1 has a uniform temperature distribution over the entire surface of the tested component with the exception of a significantly stronger heated area 10, clearly distinguished in the image of the temperature distribution pattern. Excessive heating of the area 10 is due to the increased electrical resistance of the carbon fibers in it, which is indicative of its defectiveness. The area 11, also characterized by an elevated temperature, corresponds to the spot where the inductor 3 passes at the moment of recording the temperature distribution pattern of the tested article 1.

It is obvious that the claimed method is applicable both to non-destructive testing of a carbon fiber reinforced composite material as an entire part and of its individual area. It will be appreciated that the temperature distribution pattern can be recorded both simultaneously with the process of heating the material and immediately after heating.

Detecting the presence of defective areas, in turn, may include establishing the fact of the presence / absence of defective areas and determining the location of the defective areas, if any. Though, it should be understood that in specific embodiments of the present method these two actions being related by their meaning, may not be individually included into the stages of the claimed method. For one, the articles the materials of which are tested by this method at the production stage can be automatically rejected even when it is detected that there is at least one defective area without intentional determining of its location.

Thus, the claimed invention is a method for non-destructive testing of the components produced from carbon fiber reinforced composite materials which allows for an efficient and reliable assessment of their condition over the entire depth of the material structure without exposing the coating layers to excessive load, if any.

It will be appreciated that the claimed method as defined in the appended claims is not necessarily limited to the specific features and embodiments described above. By contrast, the specific features and embodiments described above are disclosed as examples implementing the claims and other equivalent features may be covered by the claims of the present invention.

## Claims

1. A method for non-destructive testing of the components produced from carbon fiber reinforced composite materials comprising
heating the material with an external source,
recording the temperature distribution pattern of the tested material,
analyzing the recorded temperature distribution pattern and detecting the presence of defective areas,
**characterized in that** the material is heated by exposing the carbon fiber included in the material to a high-frequency electromagnetic field.

2. The method of claim 1, wherein the material is heated by a high-frequency electromagnetic field at a frequency of 50-500 kHz.

3. The method of claim 1, wherein the temperature distribution pattern is recorded by means of a thermal imaging device.
